# EUROPÄISCHE PATENTANMELDUNG

(11) **EP 0 857 730 A2**
(43) Veröffentlichungstag der Anmeldung: **12.08.1998**
(21) Anmeldenummer: 98100252.0
(22) Anmeldetag: 09.01.1998
(51) Int. Cl.: C07H 15/04, C07C 31/26, C07C 31/18, C07C 29/141

(54) **Verfahren zur Hydrierung von Zuckern**

(30) Priorität: 22.01.1997 DE 19701991
(71) Anmelder: BAYER AG, 51368 Leverkusen (DE)
(72) Erfinder: Darsow, Gerhard, Dr., 47804 Krefeld (DE)

(57) **Zusammenfassung**

Epimerenfreie Zuckeralkohole können aus den korrespondierenden Zuckern durch katalytische Hydrierung in wäßriger Lösung mit Wasserstoff hergestellt werden, wobei man die Hydrierung kontinuierlich bei einem Wasserstoffdruck von 100 bis 400 bar und einer Reaktionstemperatur von 20 bis 70°C an im Festbett angeordneten trägerfreien Formkörpern aus verpreßten Pulvern von Metallen oder Legierungen der Elemente der Eisenuntergruppe der VIII. Nebengruppe des Periodensystems mit Elementen der IV. und/oder V. Nebengruppe durchführt. Die Formkörper besitzen eine Druckfestigkeit von 20 bis 220 N und eine innere Oberfläche von 10 bis 100 m²/g.

## Beschreibung

Die Erfindung betrifft ein kostengünstiges Verfahren zur kontinuierlichen katalytischen Hydrierung von Zuckern, wie z.B. D-Xylose, α-D-Glucose, 4-O-β-D-Galactopyranosyl-α-D-glucopyranose oder 4-O-α-D-Glucopyranosyl-α-D-glucopyranose, mit Wasserstoff zu den entsprechenden Zuckeralkoholen, wie z.B. D-Xylit, D-Sorbit, 4-O-β-D-Galactopyranosyl-α-D-sorbit bzw. 4-O-α-D-Glucopyranosyl-α-D-sorbit.

Der Reaktionsverlauf läßt sich durch folgende Reaktionsschemata veranschaulichen:

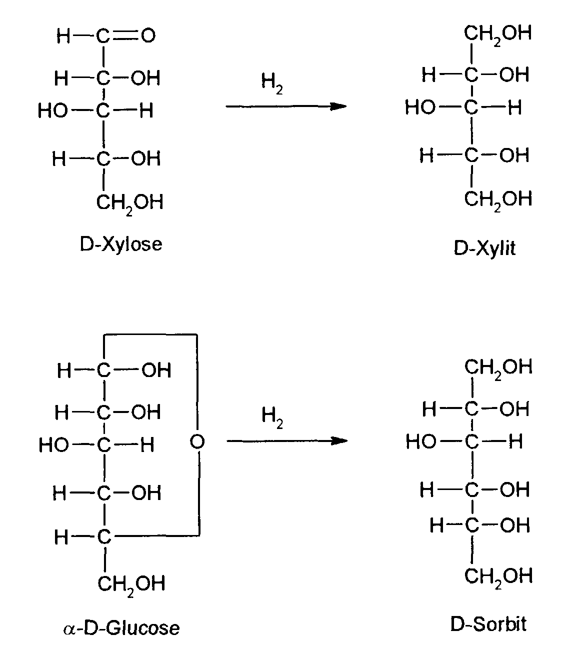

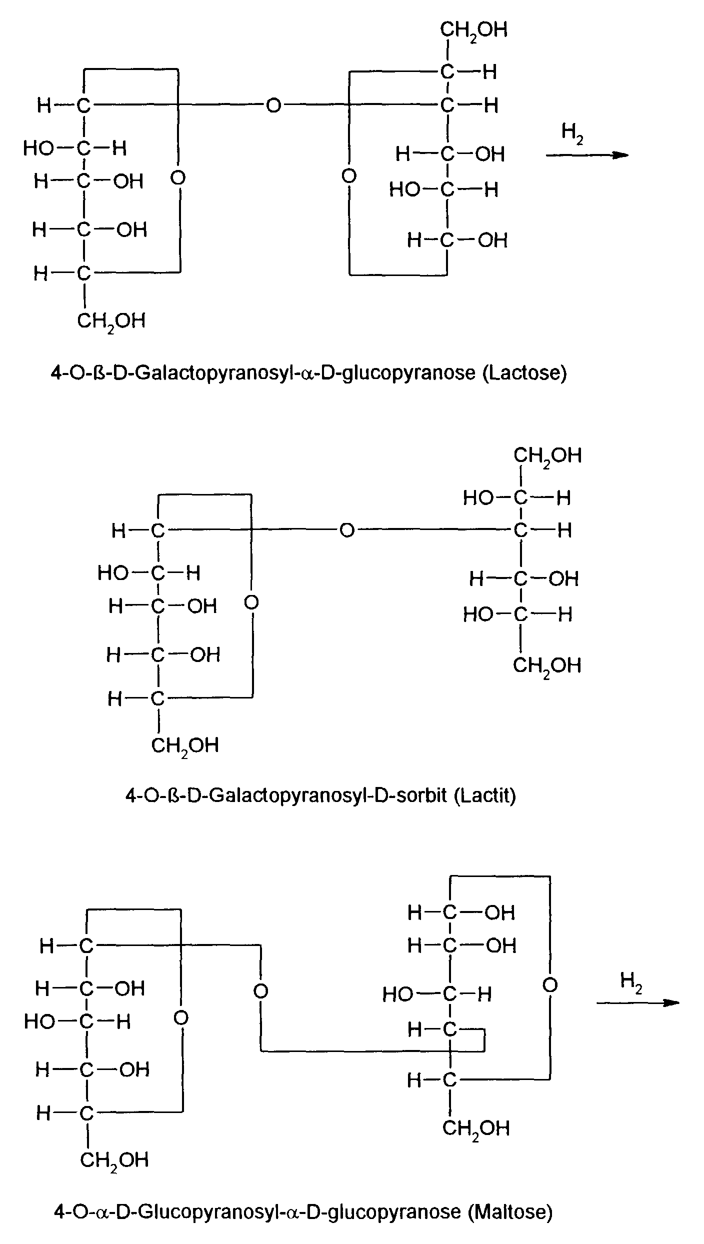

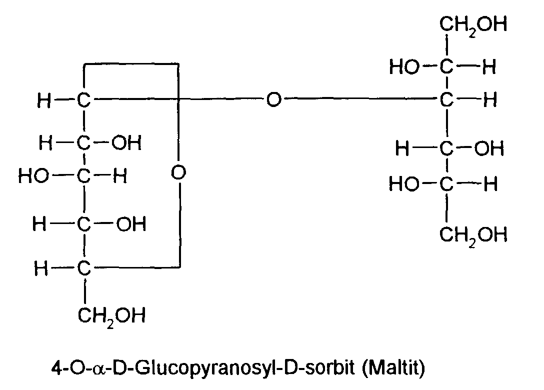

Zur Herstellung von Xylit (DE-A 19 35 934) oder Sorbit (DE-C 544 666; DE-C 554 074) wird bislang hauptsächlich ein diskontinuierliches Verfahren angewandt, bei dem ein pulverförmiger Nickelkatalysator in einem Suspensionsverfahren zum Einsatz kommt. Auch zur Herstellung des bisher in der Natur nicht nachgewiesenen Lactits ist aus EP-A 39 981 ein diskontinuierliches Verfahren bekannt, bei dem ebenfalls ein pulverförmiger Nickelkatalysator in einem Suspensionsverfahren zum Einsatz kommt. Ein solches Verfahren wird auch in US 3 741 776 zur Herstellung des Maltits vorgeschlagen.

Diskontinuierliche Suspensionsverfahren haben den Nachteil, daß ihre Kapazität, relativ zum Reaktionsvolumen, sehr klein ist und somit ein Bedarf nach großvolumigen teuren Reaktionsapparaturen und Lagertanks besteht. Der Energieverbrauch ist unökonomisch, und der Personalbedarf ist verhältnismäßig hoch. Kontinuierliche Pulverkatalysator-Verfahren, die mit zwei oder mehreren in Kaskade geschalteten diskontinuierlichen Hydrierreaktoren arbeiten, vermeiden die genannten Nachteile nur teilweise. Es bleibt der umständliche Weg, den pulverförmigen Katalysator gezielt zu dosieren, zu aktivieren, umzupumpen und quantitativ vom Reaktionsprodukt abzufiltrieren. Die Katalysatorpumpen unterliegen einer hohen mechanischen Beanspruchung. Die quantitative Entfernung des pulverförmigen Katalysators ist aufwendig (Grob- und Feinfiltrationsapparate in Wechselausführung). Ferner ist die Gefahr groß, daß der Katalysator durch die zusätzlichen Operationen verhältnismäßig schnell seine Aktivität verliert (hoher Katalysatorverbrauch). Es ist daher wünschenswert, die Reaktion über fest angeordneten Katalysatoren ablaufen zu lassen, welche eine hohe spezifische Aktivität besitzen sollten, die auch über einen längeren Zeitraum von mehreren Jahren möglichst nicht nachläßt, weil häufige Katalysatorwechsel bei Festbettreaktionen ebenfalls aufwendig wären. Auch bei fest angeordneten Katalysatoren war es bisher üblich, mehrere Reaktoren hintereinander zu schalten, wodurch sich mehrere in Serie geschaltete Reaktionszonen ergeben (DE-OS 3 214 432).

Zum Einsatz kommen hierbei überwiegend Nickelkatalysatoren auf oxidischem Trägermaterial (SiO₂/Al₂O₃) mit extrem hohen aktiven Oberflächen von 140 bis 180 m²/g, so daß die Katalysatoren in der Startphase häufig derart aktiv sind, daß sie durch zusätzliche chemische Behandlungsmethoden stabilisiert werden müssen, beispielsweise durch Sauerstoffbegasung zur Bildung monomolekularer Sauerstoffschichten auf der Katalysatoroberfläche (DE-OS 3 110 493). Die desaktivierende Stabilisierung des Katalysators macht aber dann so hohe Reaktionstemperaturen bei der Hydrierung erforderlich (130 bis 180°C), daß unkontrollierbare Nebenreaktionen möglich werden, wie Verfärbung durch Karamelisierung und hydrierende Crackung (Hydrogenolyse) der Zuckeralkohle bis zur Bildung von Methanol und sogar Methan. Außerdem gehen bei dieser Reaktionsweise stets größere Anteile von Schwermetallen in ionischer oder kolloidaler Form in Lösung, was einmal eine nachträgliche Aktivkohlebehandlung des hydrierten Produktes und zum anderen eine Entionisierung durch Ionenaustauscher erforderlich macht.

Da die meisten Hydrierverfahren mit auf pH-Werte von 7 bis 13 eingestellten Zuckerlösungen arbeiten, sind den sauren Ausgangslösungen Alkalien oder Erdalkalien beizugeben, die ebenfals wieder umständlich entfernt werden müssen (DE-OS 3 110 493; DE-OS 3 214 432). Weiterhin ist zu erwarten, daß unter den hydrierenden Bedingungen eine merkliche Epimerisierung eintritt, so daß beispielsweise aus der D-Xylose neben Xylit auch Lyxit (bzw. Arabinit und Ribit) erhalten werden. Aus α-D-Glucose wäre neben Sorbit auch Mannit zu erwarten. Ferner ist der Effekt der Spaltung der Kohlenstoffkette von Zuckern bei der katalytischen Hydrierung von Raney-Nickel bekannt; DE-OS 2 756 270 beschreibt den Effekt an einem Zuckergemisch, wie es aus der Selbstkondensation von Formaldehyd hervorgeht, wobei im Rahmen der dortigen Ausführungsbeispiele eine deutliche Verschiebung von höheren C-Kettenzahlen zu niedrigeren beobachtet wird.

Aus EP-A 423 525 ist ein Verfahren zur kontinuierlichen Hydrierung von Zuckern zu den entsprechenden epimerenfreien Zuckeralkoholen über trägerfreien Festkörpern von Elementen der Eisenuntergruppe der VIII. Nebengruppe des Periodensystem bekannt, wobei diese trägerfreien Formkörper vorzugsweise durch Verpressen und/oder Verkleben von Metallpulvern hergestellt worden sind. Hierbei ergab sich, daß die Zucker nicht nur weitgehend umgesetzt werden, sondern unter weitgehender Vermeidung von Epimerisierung und C-Kettenspaltung sowie unter Vermeidung der Bildung höhermolekularer Komponenten durch Kondensationsreaktion unter Etherbildung hauptsächlich nur jeweils ein Zuckeralkohol erhalten wird.

Aus EP-A 694 515 ist ein kostengünstigeres Verfahren zur Herstellung von Zuckeralkoholen aus der Gruppe von Xylit, Sorbit, 4-O-β-D-Galactopyranosyl-α-D-sorbit (Lactit) und 4-O-α-D-Glucopyranosyl-α-D-sorbit (Maltit) durch katalytische Hydrierung der korrespondierenden Zucker D-Xylose, α-D-Glucose, 4-O-β-D-Galactopyranosyl-α-D-glucopyranose bzw. 4-O-α-D-Glucopyranosyl-α-D-glucopyranose über trägerfreien Formkörpern von Elementen der Eisenuntergruppe der VIII. Nebengruppe des Periodensystems mit aktivierend wirkenden Elementen der VI. Nebengruppe bekannt. Es bleibt allerdings wünschenswert, die geringen Umsätze (g Zucker / l-Katalysator x h) zu erhöhen und die Katalysatorenkosten noch weiter zu senken. Außerdem ist man immer bestrebt, ein Verfahren in möglichst hoher Konzentration des zu hydrierenden Stoffes im Lösungsmittel sowie bei möglichst niedriger Temperatur durchzuführen, um auch die Energiekosten noch weiter zu senken.

Überraschenderweise wurde jetzt gefunden, daß Elemente der IV. und/oder V. Nebengruppe des Periodensystems enthaltende Metallpulver von Nickel, Kobalt und Eisen oder ihrer Legierungen nach ihrer Verpressung zu Formkörpern nicht nur ebensogut die Hydrierung der genannten Zucker zu epimerenfreien Zuckeralkoholen katalysieren, sondern daß Katalysatoren aus diesen Metallen bzw. Metall-Legierungen, die um 50 bis 70 % preiswerter sind als Katalysatoren aus den reinen Elementen der VIII.

Nebengruppe, sogar eine noch höhere Hydrieraktivität aufweisen, so daß die Hydrierreaktion bei einer bis zu 50°C niedrigeren Reaktionstemperatur durchgeführt werden kann oder aber, daß die stündliche Katalysatorbelastung um bis zu 50 % gegenüber früheren Ergebnissen erhöht werden kann. Dabei können die zum Einsatz kommenden Pulver zusätzlich gewisse Anteile (max. zulässig 20 Gew.-%) anderer nicht katalytisch wirkender Metalle oder Metall-Legierungen (z.B. Mangan, Silicium, Aluminium) enthalten, ohne daß die hohe Aktivität gemindert wird.

Gegenstand der Erfindung ist daher ein Verfahren zur Herstellung von epimerenfreien Zuckeralkoholen aus der Gruppe von Xylit, Sorbit, 4-O-β-D-Galactopyranosyl-α-D-sorbit (Lactit) und 4-O-α-D-Glucopyranosyl-α-D-sorbit (Maltit) durch katalytische Hydrierung der korrespondierenden Zucker D-Xylose, α-D-Glucose, 4-O-β-D-Galactopyranosyl-α-D-glucopyranose bzw. 4-O-α-D-Glucopyranosyl-α-D-glucopyranose in wäßriger Lösung mit Wasserstoff unter erhöhtem Druck und bei erhöhter Temperatur, das dadurch gekennzeichnet ist, daß man die Hydrierung kontinuierlich bei einem Wasserstoffdruck von 100 bis 400 bar, vorzugsweise 150 bis 300 bar und Temperaturen von 20 bis 70°C, vorzugsweise 40 bis 65°C, im Festbettverfahren in einer Reaktionszone über als Hydrierungskatalysatoren dienenden trägerfreien Formkörpern mit einer Druckfestigkeit von 20 bis 220 N, vorzugsweise 70 bis 140 N, und einer inneren Oberfläche von 10 bis 100 m²/g von
(i) einem oder mehreren Elementen der Eisenuntergruppe der VIII. Nebengruppe des Periodensystems der Elemente (Mendelejew) durchgeführt, das (die) zusätzlich mit
(ii) aktivierend wirkenden Elementen der IV. und/oder V. Nebengruppe legiert ist (sind).

Die Druckfestigkeit der trägerfreien Formkörper kann nach DIN 50 106 bestimmt werden. Die Überprüfung von trägerfreien Formkörpern auf die anspruchsgemäßen inneren Oberflächen und damit auf Brauchbarkeit für das erfindungsgemäße Verfahren können nach Methoden durchgeführt werden, die von F.M. Nelsen und F.T. Eggertsen, Analyt. Chem. 30 (1958), 1387 bzw. S.J. Gregg und S.W. Sing, Adsorption, Surface Area and Porosity, London 1982, Kap. 2 und 6, beschrieben worden sind.

Die Eisenuntergruppe der VIII. Nebengruppe des Periodensystem enthält die Elemente Eisen, Cobalt und Nickel. Die erfindungsgemäß zu verwendenden trägerfreien Formkörper enthalten eines oder mehrere dieser Metalle in Mengen von mindestens 50 Gew.-%, vorzugsweise mindestens 60 Gew.-%, insbesondere mindestens 70 Gew.-%, bezogen auf das Gesamtgewicht der trägerfreien Formkörper. Bevorzugte Eisenmetalle sind Fe und Ni.

Die IV. Nebengruppe des Periodensystems enthält die Elemente Titanium, Zirkonium und Hafnium. Die V. Nebengruppe des Periodensystems enthält die Elemente Vanadium, Niob und Tantal. Die erfindungsgemäß zu verwendenden trägerfreien Formkörper enthalten eines oder mehrere dieser Metalle in Mengen von mindestens 1,5 Gew.-%, vorzugsweise mindestens 3,0 Gew.-%, insbesondere mindestens 6,0 Gew.-%, bezogen auf das Gesamtgewicht der trägerfreien Formkörper; sie enthalten eines oder mehrere dieser Metalle in Mengen von höchstens 30 Gew.-%, vorzugsweise höchtens 20 Gew.-% und insbesondere höchstens 15 Gew.-%, bezogen auf das Gesamtgewicht der trägerfreien Formkörper. Bevorzugt unter den genannten Elementen sind Ti, Zr und V.

Die erfindungsgemäß zu verwendenden trägerfreien Formkörper können darüber hinaus - jeweils bezogen auf das Gesamtgewicht der trägerfreien Formkörper - bis zu 20 Gew.-%, vorzugsweise bis zu 15 Gew.-% anderer Metalle enthalten. Deren Untergrenze kann demnach 0 Gew.-% betragen. Beispiele solcher Metalle, die nicht katalytisch wirken müssen, umfassen Aluminium, Silicium und Mangan. Nach einer bevorzugten Ausführungsform enthalten die trägerfreien Formkörper außer den Komponenten (i) und (ii) nicht mehr als 15 Gew.-% Aluminium und/oder nicht mehr als 5 Gew.-% anderer Metalle.

Die Herstellung der trägerfreien Formkörper kann nach gebräuchlichen Methoden durch Verpressen der Metallpulver, beispielsweise auf Tablettier- oder Pelletiermaschinen, unter hohem Druck erfolgen, wobei zur Verbesserung des Haftvermögens der Metallpartikel auch Graphit und/oder Klebstoffe in Mengen von 0,5 bis 1 Gew.-%, bezogen auf das Gesamtgewicht der den Katalysator bildenden Bestandteile, zum Einsatz kommen können. Die Herstellung der trägerfreien Formkörper erfolgt vorzugsweise in einer sauerstofffreien Atmosphäre, um Oberflächenoxidation zu vermeiden. Beispiele für Formkörper sind Tabletten, Kugeln, Granulate mit Durchmessern von 2 bis 10 mm, bevorzugt 3 bis 7 mm. Tablettierte Formkörper können weiterhin zur Vergrößerung der äußeren Oberfläche mit einer axialen Durchbohrung versehen sein. Solche Formkörper haben, makroskopisch betrachtet, eine glatte Oberfläche. Die erfindungsgemäß einzusetzenden Formkörper besitzen Druckfestigkeiten von 20 bis 220 N, bevorzugt 70 bis 140 N. Dies ist wichtig, weil niedrigere Druckfestigkeiten zu einem Zerfall bzw. zu erosivem Abrieb der Formkörper führen, was eine unerwünschte Kontaminierung des Reaktionsproduktes mit Metallpulver bewirken würde.

Als Ausgangsverbindung für das erfindungsgemäße Verfahren kann kristalline D-Xylose, α-D-Glucose, α-Lactose oder α-Lactose-Monohydrat bzw. Maltose (flüssig oder in ihrer β-Form auch als kristallines Monohydrat) eingesetzt werden. Der Einsatzstoff wird vorzugsweise in sauerstofffreiem entionisiertem Wasser so gelöst, daß eine 40 bis 60 gew.-%ige, bevorzugt 45 bis 55 gew.-%ige Lösung entsteht, deren pH-Wert 4,5 bis 11,5 beträgt. Die Lösung der Monosaccharide wird bevorzugt auf einen pH-Wert von 4,5 bis 9,5, die der Disaccharide bevorzugt auf einen pH-Wert von 5,5 bis 10,5 eingestellt. Für alle Ausgangsstoffe ist der besonders bevorzugte pH-Bereich 6 bis 8,0. Die als Ausgangsverbindungen genannten Zucker zeigen, gelöst in Wasser mit einem pH-Wert von 7, eine neutrale oder, bedingt durch spurenweise Bildung von Zuckersäuren, eine schwach saure Reaktion, können aber beispielsweise durch gezielte Zugabe von basisch reagierenden wasserlöslichen Verbindungen, wie Ammoniumcarbonaten oder bevorzugt Ammoniak in wäßriger Lösung, oder von sauer reagierenden Verbindungen, wie Zuckersäuren, Sorbinsäure oder Zitronensäure, auf den gewünschten pH-Wert eingestellt werden.

Für das erfindungsgemäße Verfahren wird reiner Wasserstoff eingesetzt, der auf einen Druck von 100 bis 400 bar, bevorzugt 150 bis 300 bar, vorkomprimiert wird. Die H₂-Menge beträgt das 2-100fache der stöchiometrischen Menge, bevorzugt das 4-50fache. Die Hydrierung erfolgt kontinuierlich im Festbettverfahren an den als Hydrierkatalysatoren dienenden trägerfreien Formkörpern, indem man die zu hydrierende Lösung entweder im Gleichstrom von unten aufsteigend gemeinsam mit dem vorher zugemischten Wasserstoff über den in einem Hydrierreaktor angebrachten Katalysator strömen läßt (Gleichstromverfahren) oder aber, indem man die von unten aufsteigende zu hydrierende Lösung dem von oben einströmenden Wasserstoff entgegenführt (Gegenstromverfahren). Der Hydrierreaktor kann entweder ein einzelnes Hochdruckrohr aus Stahl oder einer Stahllegierung sein, das mit den trägerfreien Formkörpern ganz oder teilweise gefüllt wird, wobei bei gewissen Rohrquerschnitten auch die Anwendung der trägerfreien Formkörper auf Horden (Drahtkörbe oder ähnliches) nützlich sein kann, oder aber ein ummanteltes Hochdruckrohrbündel, dessen Einzelrohre mit trägerfreien Formkörpern ganz oder teilweise gefüllt werden. Weiterhin kann anstelle eines größeren Einzelrohrreaktors eine vollkontinuierliche Anordnung von mehreren kleinen Einzelreaktoren hintereinander in einer Kaskade oder parallel betrieben werden.

Die Hydrierung wird bei Temperaturen von 20 bis 70°C, bevorzugt 40 bis 65°C, durchgeführt. Niedrigere Temperaturen würden höhere Verweilzeiten oder den Verzicht auf einen weitgehend quantitativen Umsatz der Zucker ergeben. Höhere Temperaturen führen zu unkontrollierbaren Nebenreaktionen, wie Karamelisierung, Etherspaltung oder hydrierender Crackung, was zu Verfärbungen sowie Bildung unerwünschter Nebenprodukte führen kann. Die stündliche Katalysatorbelastung kann bei 250 bis 750 g der als Ausgangsverbindungen benannten Zucker pro Liter Katalysator liegen. Bei Einhaltung der genannten Reaktionsbedingungen sind bei solchen Belastungen unerwartet hohe Katalysatorstandzeiten von 20.000 Stunden und mehr zu erreichen, wobei spezifische Katalysatorverbräuche von 0,1 Gew.-% oder weniger erreicht werden.

Das erfindungsgemäße Verfahren gestattet die Herstellung der Zuckeralkohole in einer Reinheit von über 99,5 % in der Trockenmasse. Der Gehalt an nicht umgesetzten Zuckern erreicht Werte von ≤ 0,2 Gew.-%. Die handelsüblichen Spezifikationen für die Zuckeralkohole etwa nach dem Deutschen Arzneimittelbuch DAB, der United States Pharmacopeia USP oder dem Food Chemical Codex FCC, können daher mit Leichtigkeit und ohne weitere Reinigungsvorgänge direkt erfüllt werden.

Damit liegen die technischen Vorteile des erfindungsgemäßen Verfahrens außer in der durch den nahezu quantitativen Umsatz bedingten hohen Ausbeute und den ökologischen Vorteilen, die sich aus der Reinheit des hergestellten Produktes ergeben, in dem extrem niedrigen Katalysatorverbrauch, den niedrigen Katalysatorkosten, den niedrigen Energiekosten und den extrem hohen stündlichen Katalysatorbelastbarkeiten, was zu hohen Raum-Zeit-Ausbeuten führt.

Die den Reaktor verlassende wäßrige Lösung des Zuckeralkohols kann nach der Entspannung, bei welcher man den überschüssigen Wasserstoff abfangen und nach erfolgter Komprimierung und Ergänzung durch weiteren Wasserstoff erneut zum Einsatz bringen kann, bereits als Zuckeraustauschstoff in flüssiger Form Verwendung finden. Das Wasser dieser Lösung läßt sich auf verschiedene Weise, beispielsweise über Sprühtrockner, Trockenwalzen oder durch Gefriertrocknung entfernen. Als günstig hat sich erwiesen, die erhaltene, im Regelfall glasklare Lösung des Zuckeralkohols in einem Fallfilmverdampfer oder einem ähnlich arbeitenden Gerät auf einen Zuckeralkoholgehalt von etwa 70 bis 80 Gew.-% aufzukonzentrieren und anschließend nach weiterer Eindampfung in einem Vakuum-Kristallisationsgerät unter Kühlung zur teilweisen oder vollständigen Kristallisation zu bringen. Das Kristallisat läßt sich durch einen nachgeschalteten Mahlprozeß und evtl. Siebung auf eine einheitliche Korngröße bringen. Die erhaltenen Produkte sind rieselfähig.

Xylit hat einen Schmelzpunkt von 94°C. Beim Sorbit können in Abhängigkeit von den Kristallisationsbedingungen verschiedene kristalline Modifikationen anfallen, von denen die γ-Form mit einem Schmelzpunkt von 101°C die stabilste ist. In Abhängigkeit von den Kristallisationsumständen kann Lactit entweder als Dihydrat mit einem Schmelzpunkt von 78°C oder als Monohydrat mit einem Schmelzpunkt von 123°C erhalten werden. Die Löslichkeit beider Hydrate in Wasser ist unterschiedlich; das Monohydrat ist weniger löslich als das Dihydrat. Die Hydrate sind nicht hygroskopisch und zeigen daher technologische Vorteile gegenüber anderen Polyolen. Wasserfreier Lactit kann aus Lösungen in absolutem Ethanol in kristalliner Form gewonnen werden. Er schmilzt bei 146°C und ist nicht hygroskopisch. Wasserfreier Maltit kann aus Lösungen in absolutem Ethanol nach Animpfen mit Impfkristallen als kristallines Pulver gewonnen werden. Er schmilzt bei 146°C und ist hygroskopisch (J. Am. Chem. Soc. 60 (1938), 571). Alle erfindungsgemäß hergestellten Zuckeralkohole haben einen Gehalt an Katalysatorbestandteilen von unter 3 ppm; sie sind in der Regel epimerenfrei.

Als epimerenfrei im Sinne der vorliegenden Erfindung gilt ein für die Reinheit der genannten Zuckeralkohole insoweit vernachlässigbarer Gehalt an Epimeren, daß diese Zuckeralkohole die handelsüblichen Spezifikationen, wie sie im DAB sowie in der USP und im FCC angegeben sind, ohne weitere Reinigungsgänge erfüllen.

Die Zuckeralkohole werden im erfindungsgemäßen Verfahren in nahezu quantitativer Ausbeute erhalten. Dieses ist von besonderer Bedeutung, da die Entfernung (durch Etherbildung entstandener) höhermolekularer oder (durch Hydrogenolyse entstandener) niedermolekularer störender Verunreinigungen aus dem Reaktionsprodukt durch zusätzliche Reinigungsprozesse, wie Umkristallisation aus Lösungsmitteln normalerweise einen beträchtlichen ökologischen Aufwand hinsichtlich deren Entsorgung erfordert. Die dem Xylit epimeren Zuckeralkohole vom Typ des Lyxits (bzw. Arabinits und Ribits) sind im Reaktionsprodukt höchstens spurenweise (<0,1 Gew.-%) enthalten. Der zum Lactit diastereomere 4-O-β-D-Galactopyranosyl-α-D-mannit und der zum Maltit diastereomere 4-O-α-D-Glucopyranosyl-α-D-mannit sind im jeweiligen Reaktionsprodukt nicht nachweisbar.

Die erfindungsgemäß einzusetzenden sauerstofffreien und trägerfreien Festbettkatalysatoren neigen im Gegensatz zu trägerhaltigen Katalysatoren nicht zum 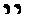 Ausbluten", d.h. nicht zum Übergang von Katalysatorbestandteilen in ionischer oder kolloidaler Form in die Lösungsphase des Substrats, so daß das Substrat nicht durch Schwermetalle kontaminiert wird, die normalerweise ebenfalls nur mühsam, beispielsweise mit Hilfe von Ionenaustauschern, aus dem Substrat entfernt werden können. Die einzusetzenden Katalysatormetalle können nach ihrem Gebrauch leicht aufgearbeitet und wiederverwendet werden, da die Schwermetalle nicht umständlich von einem Trägermaterial getrennt werden müssen. Bei Polyhydroxyverbindungen wäre weiterhin die Neigung zu befürchten, daß mit Schwermetallionen komplexe Chelatverbindungen gebildet werden, die nur schwierig aus den Zuckeralkohol-Lösungen entfernt werden können.

Die Süßkraft von Xylit erreicht etwa 80 bis 100 % der Süßkraft von Saccharose. Wegen seines angenehmen Geschmackes eignet sich Xylit als Zuckeraustauschstoff in der Diabetiker-Diät und als nicht kariogenes Süßungsmittel in Süßwaren und oralen Pharmazeutika. Für Diabetiker-Lebensmittel ist Xylit durch die Diätverordnung (Bundesgesetzblatt 1, 1982, Seite 71; zitiert nach Ullmann, 4. Aufl., Band 24, S. 777, Literaturstelle [209]) ohne Mengenbegrenzung zugelassen. Xylit ist besonders gut zur Herstellung von Süßwaren, wie Bonbons und Kaugummi, geeignet (Swiss Dent. 1, (7/8) 1980, Seite 25 bis 27; zitiert nach Ullmann, 4. Aufl., Band 24, S. 777, Literaturstelle [223]). Auch Produkte zur Behandlung des Mund- und Rachenraumes, wie Zahnpasten, rachendesinfizierende Tabletten, Hustenbonbons, werden zunehmend mit Xylit gesüßt (Swiss Dent. 3, (7/8) 1982, Seite 25 bis 30; zitiert nach Ullmann, 4. Aufl., Band 24, S. 777, Literaturstelle [224]).

Die Süßkraft von Sorbit erreicht etwa 50 bis 60 % der Süßkraft von Saccharose. Zur Erhöhung der Süßkraft kann die wäßrige Lösung mit künstlichen Süßstoffen, beispielsweise mit Cyclohexylsulfamat oder Asparaginsäure-phenylalanin-methylester, versetzt und durch gemeinsame Vakuumkristallisation in kristalliner Form gewonnen werden. Man kann aber auch die künstlichen Süßstoffe in fester Form mit dem Sorbit-Kristallisat vermischen. Sorbit kann auch in flüssiger oder fester Form mit anderen süß schmeckenden Zuckeralkoholen, beispielsweise mit Xylit, Maltit, Lactit und anderen, vermischt werden. Sorbit wird im menschlichen Körper nur in untergeordnetem Maße resorbiert und nur in diesem Anteil abgebaut. Daher ist Sorbit als Zuckerersatzstoff für Diabetiker und als kalorienarmer Süßstoff geeignet. Ferner ist er weniger kariogen als Saccharose oder andere Zucker.

Lactit wird im menschlichen Körper nicht als Kohlehydrat abgebaut und im Dünndarm weder hydrolysiert noch resorbiert. Daher ist Lactit als Zuckerersatzstoff für Diabetiker geeignet. Ferner ist er weniger kariogen als Saccharose. Die Süßkraft von Lactit erreicht etwa 40 % der Süßkraft von Saccharose. Zur Erhöhung der Süßkraft kann die wäßrige Lösung wie bei Sorbit mit künstlichen Süßstoffen versetzt und durch gemeinsame Vakuumkristallisation in kristalliner Form gewonnen werden. Man kann aber auch die künstlichen Süßstoffe in fester Form mit dem Lactit-Kristallisat vermischen. Lactit kann außerdem in flüssiger oder fester Form mit anderen süß schmeckenden Zuckeralkoholen, beispielsweise mit Sorbit, Xylit und anderen, vermischt werden.

Maltit wird im menschlichen Körper durch amylolytische Enzyme nur schwer abgebaut. Daher ist Maltit als Zuckerersatzstoff für kalorienreduzierte Diät und für Diabetiker geeignet (Ullmanns Encyclopädie der technischen Chemie, 4. Auflage, Band 24, Weinheim 1983, S. 771). Die Süßkraft von Maltit erreicht die Süßkraft von Saccharose. Maltit kann in flüssiger Form mit anderen süßschmeckenden Zuckeralkoholen, beispielsweise mit Sorbit, Xylit und anderen, vermischt werden. Besonders der Einsatz in der Getränkeindustrie empfiehlt sich wegen seiner hohen Süßkraft und seiner geringen Neigung zur Kristallisation selbst in hohen Konzentrationen.

Toxische Effekte von Xylit, Sorbit und Lactit konnten auch in Langzeitstudien nicht festgestellt werden (Ullmanns Encyclopädie der technischen Chemie, 4. Auflage, Band 24, Weinheim 1983, S. 779), so daß sich vielseitige Anwendungsmöglichkeiten im Lebensmittelbereich, bei der Herstellung von Diabetikerprodukten sowie von zuckerfreien Süßigkeiten und Nahrungsmitteln mit geringem Nährwert ergeben. Auch bei Maltit konnten in Langzeitstudien keine toxischen Effekte festgestellt werden.

### Beispiele

Die Prozentangaben der nachfolgenden Beispiele beziehen sich auf das Gewicht.

### Beispiel 1

Ein senkrecht stehendes, wärmeisoliertes Hochdruckrohr aus nichtrostendem Stahl von 45 mm Innendurchmesser und 1 m Länge wurde mit 1,4 l eines durch Tablettierung eines Metallpulvers aus einer Ni/Zr-Legierung mit einem Zr-Gehalt von 12,8 % hergestellten Hydrierungskatalysators gefüllt, der bei einer Zylinderhöhe von 5 mm und einem Durchmesser von 5 mm eine Druckfestigkeit von 109 N auf die Zylindermantelfläche und eine innere Oberfläche von 74 m²/g aufwies. Durch dieses Rohr wurden gemeinsam mit der fünffach molaren Menge von unter einem Druck von 300 bar stehendem, hochreinem Wasserstoff stündlich 450 ml einer 45 %igen Lösung von D-Xylose in entionisiertem sauerstofffreiem Trinkwasser mit einem pH-Wert von 7,0 kontinuierlich von unten nach oben aufsteigend gepumpt.

Wäßrige Lösung und Wasserstoff wurden vorab gemeinsam durch einen Wärmeaustauscher geführt und so hoch erhitzt, daß sie in das Hochdruckrohr mit einer Temperatur von 60°C eintraten. Das das Hochdruckrohr verlassende Gemisch von wäßriger Lösung und überschüssigem Wasserstoff wurde über einen Kühler in einen Abscheider geführt, von wo der Wasserstoff nach Ersatz der verbrauchten Menge wieder zusammen mit frischer D-Xylose-Lösung in den Vorwärmer und von da erneut in das Hochdruckrohr gepumpt wurde. Die farblose und klare wäßrige Lösung wurde entspannt und in einem Fallfilmverdampfer auf einen Zuckeralkoholgehalt von ca. 75 % aufkonzentriert und anschließend nach weiterer Eindampfung in einem Vakuumkristaller unter Kühlung zur Kristallisation gebracht. Man erhielt ein weißes, leicht hygroskopisches, geruchloses Festprodukt, das zu einem feinkristallinen Pulver verarbeitet wurde. Der gebildete Xylit war ansonsten hochrein und zeigte in der stabilen rhombischen Kristallform einen Schmelzpunkt von 94°C. Der Gehalt an nicht hydrierter D-Xylose lag bei ≤0,1 %. Ni- und Zr-Gehalt lagen jeweils bei <1 ppm. Der Katalysator war auch nach einer Laufzeit von 1852 Stunden unverändert wirksam.

### Beispiel 2

Durch ein Hochdruckrohr wie in Beispiel 1, aber aus Hochdruckstahl N9, wurde bei einer Temperatur von 60°C und einem Wasserstoffdruck von 300 bar der Wasserstoff im umgekehrten Reaktionsfluß wie in Beispiel 1 der aufsteigenden Lösung von D-Xylose entgegengeführt, wobei stündlich eine gleich große Menge einer 45 %igen wäßrigen Lösung von D-Xylose hydriert wurde, die einen pH-Wert von 7,0 aufwies. Der Katalysator war durch Tablettierung von Metallpulver aus einer Ni/Zr-Legierung mit einem Zr-Gehalt von 14,9 %, die zusätzlich mit einem Al-Anteil von 10,5 % legiert war, hergestellt worden. Die Tabletten hatten bei einer Zylinderhöhe von 5 mm und einem Durchmesser von 5 mm eine Druckfestigkeit von 75 N auf die Zylindermantelfläche und eine innere Oberfläche von 81 m²/g. Nach einer Laufzeit von 2224 Stunden mit unverminderter Wirksamkeit lag der Umsatz der D-Xylose bei ≥99,8 %. Der Gehalt an nicht hydrierter D-Xylose im auskristallisierten Xylit, der einen Reinheitsgrad von ≥99,6 % aufwies, betrug 0,1 %. Ni-, Zr- und Al-Gehalt lagen jeweils bei 2 ppm.

### Beispiel 3

In einem Hochdruckrohr wie in Beispiel 1 wurde bei einer Temperatur von 60°C und einem Wasserstoffdruck von 300 bar in gleicher Weise wie in Beispiel 1 stündlich eine gleich große Menge einer 45 %igen wäßrigen Lösung von D-Xylose hydriert, die einen pH-Wert von 7,5 aufwies. Der Katalysator war durch Tablettierung einer Ni/Fe/Zr-Legierung gewonnen worden. Die Legierung enthielt einen Fe-Anteil in Ni von 5 % sowie einen Zr-Anteil von 10,9 %. Die Tabletten hatten bei einer Zylinderhöhe von 5 mm und einem Durchmesser von 5 mm eine Druckfestigkeit von 107 N auf die Zylindermantelfläche und eine innere Oberfläche von 93 m²/g. Der in einem Vakuumkristaller unter Animpfen mit Impfkristallen gewonnene kristalline Xylit hatte einen Reinheitsgrad von ≥99,6 %. Der Gehalt an nicht umgesetzter D-Xylose lag bei 0,1 %. Ni-, Fe- und Zr-Gehalt lagen jeweils bei <1 ppm. Der Katalysator war nach einer Laufzeit von 1620 Stunden noch unverändert wirksam.

### Beispiel 4

In einem Hochdruckrohr wie in Beispiel 1 wurden bei einer Temperatur von 60°C und einem Wasserstoffdruck von 300 bar in gleicher Weise wie in Beispiel 1 stündlich 400 ml einer 45 %igen wäßrigen Lösung von D-Xylose hydriert, die einen pH-Wert von 6,5 aufwies. Der Katalysator wurde durch Tablettierung einer pulverisierten Ni/Zr-Legierung und einer pulverisierten Ni/Ti-Legierung hergestellt und hatte einen Zr-Gehalt von 12,1 % und einen Ti-Gehalt von 5,8 %. Die Tabletten hatten bei einer Zylinderhöhe von 5 mm und einem Durchmesser von 5 mm eine Druckfestigkeit von 103 N auf die Zylindermantelfläche und eine innere Oberfläche von 81 m²/g. Der in einem Vakuumdrehrohr gewonnene Xylit zeigte einen Gehalt an nicht umgesetzter D-Xylose von ≤0,1 %. Der Ni-, Zr-, Ti-Gehalt lagen jeweils bei <1 ppm. Der Katalysator war nach einer Laufzeit von 1140 Stunden noch unvermindert wirksam.

### Beispiel 5

Ein Hochdruckrohr wie in Beispiel 1 wurde mit 1,4 l eines durch Tablettierung von Metallpulver aus einer Ni/Zr/Al-Legierung mit einem Zr-Gehalt von 14,9 % hergestellten Hydrierungskatalysators gefüllt, der bei einer Zylinderhöhe von 5 mm und einem Durchmesser von 5 mm eine Druckfestigkeit von 75 N auf die Zylindermantelfläche und eine innere Oberfläche von 81 m²/g aufwies. Durch dieses Rohr wurden gemeinsam mit der zehnfach molaren Menge von unter einem Druck von 300 bar stehendem hochreinem Wasserstoff stündlich 450 ml einer 45 %igen Lösung von α-D-Glucose in entionisiertem sauerstofffreiem Trinkwasser mit einem pH-Wert von 7,0 kontinuierlich von unten nach oben aufsteigend gepumpt. Wäßrige Lösung und Wasserstoff wurden vorher gemeinsam durch einen Wärmeaustauscher geführt und so hoch erhitzt, daß sie in das Hochdruckrohr mit einer Temperatur von 60°C eintraten. Das das Hochdruckrohr verlassende Gemisch von wäßriger Lösung und überschussigem Wasserstoff wurde über einen Kühler in einen Abscheider geführt, von wo Wasserstoff nach Ersatz der verbrauchten Menge wieder zusammen mit frischer α-D-Glucose-Lösung in den Vorwärmer und von da erneut in das Hochdruckrohr gepumpt wurde. Die farblose und klare wäßrige Lösung wurde entspannt und in einem Fallfilmverdampfer auf einen Zuckeralkoholgehalt von ca. 70 % aufkonzentriert und anschließend nach weiterer Eindampfung in einem Vakuumkristaller unter Kühlung zur Kristallisation gebracht. Es wurde ein weißes, leicht hygroskopisches, geruchloses Festprodukt erhalten, das zu einem feinkristallinen Pulver vermahlen wurde. Der gebildete Sorbit war ansonsten hochrein und zeigte in der stabilen γ-Form einen Schmelzpunkt von 101°C. Der Gehalt an nicht hydrierter α-D-Glucose lag bei ≤0,1 % Ni-, Zr- und Al-Gehalt lagen jeweils bei <1 ppm. Der Katalysator war auch nach einer Laufzeit von 2622 Stunden unverändert wirksam.

### Beispiel 6

Durch ein Hochdruckrohr wie in Beispiel 1 wurde bei einer Temperatur von 60°C und einem Wasserstoffdruck von 300 bar der Wasserstoff im umgekehrten Reaktionsfluß, wie in Beispiel 1 beschrieben, der aufsteigenden Lösung von α-D-Glucose entgegengeführt, wobei stündlich eine gleich große Menge 45 %iger wäßriger Lösung von α-D-Glucose wie in Beispiel 1 hydriert wurde, die einen pH-Wert von 7,0 aufwies. Der Katalysator war durch Tablettierung eines Metallpulvers aus einer Ni/Zr/Al-Legierung mit einem Zr-Gehalt von 14,8 % und einem Al-Anteil von 10,5 % hergestellt worden. Die Tabletten hatten bei einer Zylinderhöhe von 5 mm und einem Durchmesser von 5 mm eine Druckfestigkeit von 75 N auf die Zylindermantelfläche und eine innere Oberfläche von 81 m²/g. Nach einer Laufzeit von 1842 Stunden mit unverminderter Wirksamkeit lag der Umsatz der α-D-Glucose bei 99,9 %. Der Gehalt an nicht hydrierter α-D-Glucose im auskristallisierten Sorbit, der einen Reinheitsgrad von ≥99,7 % aufwies, betrug ≤0,1 %. Der Ni-Gehalt lag bei <1 ppm. Der Zr- und Al-Gehalt lagen jeweils bei <2 ppm.

### Beispiel 7

In einem Hochdruckrohr wie in Beispiel 1 wurde bei einer Temperatur von 55°C und einem Wasserstoffdruck von 300 bar in gleicher Weise wie in Beispiel 1 stündlich eine gleich große Menge einer 40 %igen wäßrigen Lösung von α-D-Glucose hydriert, die einen pH-Wert von 7,5 aufwies. Der Katalysator war durch Tablettierung einer pulverisierten Ni/Fe/Zr-Legierung gewonnen worden. Die Legierung enthielt einen Fe-Anteil in Ni von 5 % sowie einen Zr-Anteil von 10,9 %. Die Tabletten hatten bei einer Zylinderhöhe von 5 mm und einem Durchmesser von 5 mm eine Druckfestigkeit von 107 N auf die Zylindermantelfläche und eine innere Oberfläche von 93 m²/g. Der in einem Vakuumkristaller gewonnene kristalline Sorbit hatte einen Reinheitsgrad von ≥99,6 %. Der Gehalt an nicht umgesetzter α-D-Glucose lag bei 0,1 %. Ni-, Zr- und Fe-Gehalt lagen jeweils bei <1 ppm. Der Katalysator war nach einer Laufzeit von 1448 Stunden noch unverändert wirksam.

### Beispiel 8

In einem Hochdruckrohr wie in Beispiel 1 wurden bei einer Temperatur von 60°C und einem Wasserstoffdruck von 300 bar in gleicher Weise wie in Beispiel 1 stündlich 400 ml einer 45 %igen wäßrigen Lösung von α-D-Glucose hydriert, die einen pH-Wert von 6,5 aufwies. Der Katalysator war durch Tablettierung von Metallpulver einer Ni/Zr/V-Legierung mit einem Zr-Gehalt von 10,9 % und einem V-Gehalt von 3,4 %, die zusätzlich einen Al-Gehalt von 10,1 % aufwies, hergestellt worden. Die Tabletten hatten bei einer Zylinderhöhe von 5 mm und einem Durchmesser von 5 mm eine Druckfestigkeit von 109 N auf die Zylindermantelfläche und eine innere Oberfläche von 81 m²/g. Der in einem Vakuumdrehrohr gewonnene Sorbit zeigte einen Gehalt an nicht umgesetzter α-D-Glucose von ≤0,1 %. Ni-, Zr-, V- und Al-Gehalt lagen jeweils bei 1 ppm. Der Katalysator war nach einer Laufzeit von 1662 Stunden noch unvermindert wirksam.

### Beispiel 9

Ein Hochdruckrohr wie in Beispiel 1 wurde mit 1,4 l eines durch Tablettierung von Metallpulver aus einer Ni/Zr-Legierung mit einem Zr-Anteil von 14,9 % hergestellten Hydrierungskatalysators gefüllt, der bei einer Zylinderhöhe von 3 mm und einem Durchmesser von 3 mm eine Druckfestigkeit von 85 N auf die Zylindermantelfläche und eine innere Oberfläche von 85 m²/g aufwies. Durch dieses Rohr wurden gemeinsam mit der zehnfach molaren Menge von unter einem Druck von 300 bar stehendem, hochreinem Wasserstoff stündlich 500 ml einer 40 %igen Lösung von 4-O-β-D-Galactopyranosyl-α-D-glucopyranose in entionisiertem sauerstofffreiem Trinkwasser mit einem pH-Wert von 7,0 kontinuierlich von unten nach oben aufsteigend gepumpt. Wäßrige Lösung und Wasserstoff wurden vorher gemeinsam über einen Wärmetauscher geführt und so hoch erhitzt, daß sie in das Hochdruckrohr mit einer Temperatur von 60°C eintraten. Das das Hochdruckrohr verlassende Gemisch von wäßriger Lösung und überschüssigem Wasserstoff wurde über einen Kühler in einen Abscheider geführt, von wo der Wasserstoff nach Ersatz der verbrauchten Menge wieder zusammen mit frischer 4-O-β-D-Galactopyranosyl-α-D-glucopyranose in den Vorwärmer und von da erneut in das Hochdruckrohr gepumpt wurde. Die farblose und klare wäßrige Lösung wurde entspannt und in einem Fallfilmverdampfer auf einen Zuckeralkoholgehalt von ca. 80 % aufkonzentriert und anschließend nach weiterer Eindampfung in einem Vakuumkristaller unter Kühlung zur Kristallisation gebracht. In Abhängigkeit von den Kristallisationsumständen und dem Restwassergehalt der eingedampften Lösung ließ sich dabei entweder das Dihydrat mit einem Schmelzpunkt von 77 bis 78°C oder das Monohydrat mit einem Schmelzpunkt von 122 bis 123°C isolieren; der gebildete 4-O-β-D-Galactopyranosyl-α-D-sorbit war ansonsten rein (Reinheitsgrad ≥99,7%). Der Gehalt an nicht hydrierter 4-O-β-D-Galactopyranosyl-α-D-glucopyranose lag bei ≤0,1 %. Der Gehalt an Sorbit betrug ≤0,1 %. 4-O-β-D-Galactopyranosyl-D-mannit bzw. Mannit konnten nicht nachgewiesen werden. Ni-Gehalt und Zr-Gehalt lagen jeweils bei 1 ppm. Der Katalysator war auch nach einer Laufzeit von 1880 Stunden unverändert wirksam.

### Beispiel 10

Durch ein Hochdruckrohr wie in Beispiel 1 wurde bei einer Temperatur von 65°C und einem Wasserstoffdruck von 250 bar der Wasserstoff im umgekehrten Reaktionsfluß, wie in Beispiel 1 beschrieben, der aufsteigenden Lösung von 4-O-β-D-Galactopyranosyl-α-D-glucopyranose entgegengeführt, wobei stündlich eine gleich große Menge 40 %iger wäßriger Lösung von 4-O-β-D-Galactopyranosyl-α-D-glucopyranose wie in Beispiel 1 hydriert wurde, die einen pH-Wert von 6,5 aufwies. Der Katalysator wurde durch Tablettierung aus Metallpulver einer Ni/Zr/Al-Legierung mit einem Zr-Anteil von 10,9 % und einem Al-Anteil von 10,5 % hergestellt. Die Tabletten hatten bei einer Zylinderhöhe von 5 mm und einem Durchmesser von 5 mm eine Druckfestigkeit von 78 N auf die Zylindermantelfläche und eine innere Oberfläche von 81 m²/g. Nach einer Laufzeit von 1912 Stunden mit unverminderter Wirksamkeit lag der Gehalt an 4-O-β-D-Galactopyranosyl-α-D-sorbit des in einem Rotationsverdampfer zur Trockne eingedampften Reaktionsgemisches bei 99,6 %. Der Gehalt an nicht hydrierter 4-O-β-D-Galactopyranosyl-α-D-glucopyranose betrug ≤0,1 %. Der Gehalt an Sorbit lag bei 0,1 %. 4-O-β-D-Galactopyranosyl-D-mannit bzw. Mannit konnten nicht nachgewiesen werden. Ni-, Zr- und Al-Gehalt lagen jeweils bei <3 ppm.

### Beispiel 11

In einem Hochdruckrohr wie in Beispiel 1 wurde bei einer Temperatur von 65°C und einem Wasserstoffdruck von 300 bar in gleicher Weise wie in Beispiel 1 stündlich eine gleich große Menge einer 40 %igen wäßrigen Lösung von 4-O-β-D-Galactopyranosyl-α-D-glucopyranose hydriert, die einen pH-Wert von 7,5 aufwies. Der Katalysator wurde durch Tablettierung einer pulverisierten Ni/Zr/V/Al-Legierung gewonnen. Die Legierung enthielt einen Zr-Anteil von 10,9 %, einen V-Gehalt von 3,4 % und einen Al-Anteil von 10,1 %. Die Tabletten hatten bei einer Zylinderhöhe von 5 mm und einem Durchmesser von 5 mm eine Druckfestigkeit von 109 N auf die Zylindermantelfläche und eine innere Oberfläche von 81 m²/g. Der in einem Vakuumkristaller gewonnene 4-O-β-D-Galactopyranosyl-α-D-sorbit hatte einen Reinheitsgrad von ≥99,6 %. Der Gehalt an nicht umgesetzter 4-O-β-D-Galactopyranosyl-α-D-glucopyranose lag bei 0,1 %. Der Sorbitgehalt betrug 0,1 %. 4-O-β-D-Galactopyranosyl-D-mannit bzw. Mannit konnten nicht nachgewiesen werden. Ni-, Zr-, V-, Al-Gehalt lagen jeweils bei <2 ppm. Der Katalysator war nach einer Laufzeit von 2016 Stunden noch unvermindert wirksam.

### Beispiel 13

Ein Hochdruckrohr wie in Beispiel 1 wurde mit 1,4 l eines durch Tablettierung von Metallpulver aus einer Legierung von Ni/Zr/Ti mit einem Zr-Gehalt von 8,1 % und einem Ti-Gehalt von 5,8 % hergestellten Hydrierungskatalysators gefüllt, der bei einer Zylinderhöhe von 5 mm und einem Durchmesser von 5 mm eine Druckfestigkeit von 103 N auf die Zylindermantelfläche und eine innere Oberfläche von 81 m²/g aufwies. Durch dieses Rohr wurden gemeinsam mit der zehnfach molaren Menge von unter einem Druck von 300 bar stehendem, hochreinem Wasserstoff stündlich 400 ml einer 50 %igen wäßrigen Lösung von 4-O-α-D-Glucopyranosyl-α-D-glucopyranose in entionisiertem sauerstofffreiem Trinkwasser mit einem pH-Wert von 7,0 kontinuierlich von unten nach oben aufsteigend gepumpt. Wäßrige Lösung und Wasserstoff wurden vorab gemeinsam durch einen Wärmeaustauscher geführt und so hoch erhitzt, daß sie in das Hochdruckrohr mit einer Temperatur von 60°C eintraten. Das das Hochdruckrohr verlassende Gemisch von wäßriger Lösung und überschüssigem Wasserstoff wurde über einen Abscheider geführt, von wo der Wasserstoff nach Ersatz der verbrauchten Menge wieder zusammen mit frischer 4-O-α-D-Glucopyranosyl-α-D-glucopyranose in den Vorwärmer und von da erneut in das Hochdruckrohr gepumpt wurde. Die farblose und klare wäßrige Lösung wurde entspannt und in einem Fallfilmverdampfer auf einen Zuckeralkoholgehalt von 80 % aufkonzentriert und anschließend nach weiterer Eindampfung in einem Vakuumkristaller unter Kühlung und gegebenenfalls Hinzufügung von Impfkristallen zur Kristallisation gebracht. Der kristalline 4-O-α-D-Glucopyranosyl-α-D-sorbit zeigte einen Reinheitsgrad von ≥99,6 %. Der Gehalt an nicht hydrierter 4-O-α-D-Glucopyranosyl-α-D-glucopyranose lag bei ≤0,1 %. Der Gehalt an Sorbit betrug ≤0,1 %. Mannit konnte nicht nachgewiesen werden. Der Katalysator war auch nach einer Laufzeit von 1612 Stunden unverändert wirksam.

### Beispiel 14

Durch ein Hochdruckrohr wie in Beispiel 1 wurde bei einer Temperatur von 65°C und einem Wasserstoffdruck von 250 bar der Wasserstoff im umgekehrten Reaktionsfluß wie in Beispiel 1 der aufsteigenden Lösung von 4-O-α-D-Glucopyranosyl-α-D-glucopyranose entgegengeführt, wobei stündlich eine gleich große Menge 45 %iger wäßriger Lösung von 4-O-α-D-Glucopyranosyl-α-D-glucopyranose wie in Beispiel 1 hydriert wurde, die einen pH-Wert von 6,5 aufwies. Der Katalysator wurde durch Tablettierung von Ni/Zr/Al-Pulver mit einem Zr-Gehalt von 14,9 % und einem Al-Anteil von 10,5 % hergestellt. Die Tabletten hatten bei einer Zylinderhöhe von 5 mm und einem Durchmesser von 5 mm eine Druckfestigkeit von 75 N auf die Zylindermantelfläche und eine innere Oberfläche von 81 m²/g. Nach einer Laufzeit von 1418 Stunden mit unverminderter Wirksamkeit lag der Gehalt an 4-O-α-D-Glucopyranosyl-α-D-sorbit des in einem Rotationsverdampfer zur Trockne eingedampften Reaktionsgemisches bei 99,8 %. Der Gehalt an nicht hydrierter 4-O-α-D-Glucopyranosyl-α-D-glucopyranose betrug ≤0,1 %. Der Gehalt an Sorbit lag bei ≤0,1 %.

### Beispiel 15

In einem Hochdruckrohr wie in Beispiel 1 wurde bei einer Temperatur von 60°C und einem Wasserstoffdruck von 300 bar in gleicher Weise wie in Beispiel 1 stündlich eine gleich große Menge einer 45 %igen wäßrigen Lösung von 4-O-α-D-Glucopyranosyl-α-D-glucopyranose hydriert, die einen pH-Wert von 7,5 aufwies. Der Katalysator wurde durch Tablettierung einer pulverisierten Ni/Fe/Zr-Legierung mit einem Fe-Anteil von 5 % und einem Zr-Gehalt von 10,9 % hergestellt. Die Tabletten hatten bei einer Zylinderhöhe von 5 mm und einem Durchmesser von 5 mm eine Druckfestigkeit von 103 N auf die Zylindermantelfläche und eine innere Oberfläche von 95 m²/g. Der in einem Vakuumverdampfer gewonne 4-O-α-D-Glucopyranosyl-α-D-sorbit hatte einen Reinheitsgrad von 99,7 %. Der Gehalt an nicht umgesetzter 4-O-α-D-Glucopyranosyl-α-D-glucopyranose lag bei 0,1 %. Der Sorbitgehalt betrug 0,2 %. Ni-, Fe- und Zr-Gehalt lagen bei <3 ppm. Der Katalysator war nach einer Laufzeit von 1604 Stunden unverändert wirksam.

### Beispiel 16

In einem Hochdruckrohr wie in Beispiel 1 wurde bei einer Temperatur von 65°C und einem Wasserstoffdruck von 200 bar in gleicher Weise wie in Beispiel 1 eine ebenso große Menge einer 40 %igen wäßrigen Lösung von 4-O-α-D-Glucopyranosyl-α-D-glucopyranose hydriert, die einen pH-Wert von 6,5 aufwies. Der Katalysator wurde durch Tablettierung eines Metallpulvers aus einer Ni/Zr/V/Al-Legierung mit einem Zr-Gehalt von 14,9 %, einem V-Gehalt von 6,4 % und einem Al-Gehalt von 10,4 % gewonnen. Die Tabletten hatten bei einer Zylinderhöhe von 5 mm und einem Durchmesser von 5 mm eine Druckfestigkeit von 95 N auf die Zylindermantelfläche und eine innere Oberfläche von 88 m²/g. Der in einem Vakuumdrehrohr gewonnene 4-O-α-D-Glucopyranosyl-α-D-sorbit hatte einen Reinheitsgrad von ≥99,6 %. Der Gehalt an nicht umgesetzter 4-O-α-D-Glucopyranosyl-α-D-glucopyranose lag bei ≤0,1 %. Der Sorbitgehalt betrug 0,1 %. Der Katalysator war nach einer Laufzeit von 3128 Stunden unvermindert wirksam.

## Patentansprüche

1. Verfahren zur Herstellung von epimerenfreien Zuckeralkoholen aus der Gruppe von Xylit, Sorbit, 4-O-β-D-Galactopyranosyl-α-D-sorbit und 4-O-α-D-Glucopyranosyl-α-D-sorbit durch katalytische Hydrierung der korrespondierenden Zucker D-Xylose, α-D-Glucose, 4-O-β-D-Galactopyranosyl-α-D-glucopyranose bzw. 4-O-α-D-Glucopyranosyl-α-D-glucopyranose in wäßriger Lösung mit Wasserstoff unter erhöhtem Druck und bei erhöhter Temperatur, dadurch gekennzeichnet, daß man die Hydrierung kontinuierlich bei einem Wasserstoffdruck von 100 bis 400 bar und Temperaturen von 20 bis 70°C im Festbettverfahren in einer Reaktionszone über als Hydrierungskatalysatoren dienenden Formkörpern mit einer Druckfestigkeit von 20 bis 220 N und einer inneren Oberfläche von 10 bis 100 m²/g von
(i) einem oder mehreren Elementen der Eisenuntergruppe der VIII. Nebengruppe des Periodensystems der Elemente (Mendeljew) durchführt, das (die) zusätzlich mit
(ii) aktivierend wirkenden Elementen der IV. und/oder V. Nebengruppe legiert ist (sind).

2. Verfahren nach Anspruch 1, dadurch gekennzeichnet, daß die Formkörper zylinder- oder kugelförmig sind und Durchmesser von 2 bis 10 mm, bevorzugt 3 bis 7 mm besitzen.

3. Verfahren nach Anspruch 1, dadurch gekennzeichnet, daß man die Hydrierung der Zucker in 40 bis 60 %iger wäßriger Lösung bei einem pH-Wert von 4,5 bis 11,5 durchführt.

4. Verfahren nach Anspruch 1, dadurch gekennzeichnet, daß die als Katalysatoren dienenden Formkörper eine Druckfestigkeit von 70 bis 140 N haben.

5. Verfahren nach Anspruch 1, dadurch gekennzeichnet, daß bei einem Wasserstoffdruck von 150 - 300 bar gearbeitet wird.

6. Verfahren nach Anspruch 1, dadurch gekennzeichnet, daß bei einer Temperatur von 40 bis 65°C gearbeitet wird.

7. Verfahren nach Anspruch 1, dadurch gekennzeichnet, daß die als Katalysatoren dienenden Formkörper eines oder mehrere Metalle der Eisenuntergruppe zu mindestens 50 Gew.-%, bevorzugt zu mindestens 60 Gew.-%, besonders bevorzugt zu mindestens 70 Gew.-%, bezogen auf das Gesamtgewicht der Formkörper, enthalten.

8. Verfahren nach Anspruch 1, dadurch gekennzeichnet, daß die als Katalysatoren dienenden Formkörper eines oder mehrere Metalle der IV. und V. Nebengruppe des Periodensystems zu mindestens 1,5 Gew.-%, bevorzugt zu mindestens 3,0 Gew.-%, besonders bevorzugt zu mindestens 6,0 Gew.-%, bezogen aufdas Gesamtgewicht der Formkörper, enthalten.

9. Verfahren nach Anspruch 1, dadurch gekennzeichnet, daß die als Katalysatoren dienenden Formkörper eines oder mehrere Metalle der IV. und V. Nebengruppe zu höchstens 30 Gew.-%, bevorzugt zu höchstens 20 Gew.-%, besonders bevorzugt zu höchstens 15 Gew.-%, bezogen auf das Gesamtgewicht der Formkörper, enthalten.

10. Verfahren nach Anspruch 1, dadurch gekennzeichnet, daß die als Katalysatoren dienenden Formkörper eines oder mehrere nicht katalytisch wirksame Metalle in einer Menge von höchstens 20 Gew.-%, bevorzugt von höchstens 15 Gew.-%, bezogen auf das Gesamtgewicht der Formkörper enthalten.
